# EUROPEAN PATENT SPECIFICATION

(11) **EP 0 370 458 B1**
(45) Date of publication and mention of the grant of the patent: **15.01.1997**
(21) Application number: 89121513.9
(22) Date of filing: 21.11.1989
(51) Int. Cl.: C07K 14/00, C12N 15/62, G01N 33/569

(54) **Synthetic DNA derived recombinant HIV-1 antigens**
Von synthetischer DNS abgeleitete rekombinante HIV-1-Antigene
Antigènes à partir d'ADN synthétique dérivé du virus de l'immunodéficience humaine (HIV-1) recombinant

(30) Priority: 23.11.1988 US 275309
(43) Date of publication of application: 30.05.1990
(60) Divisional application: 95114545.7
(73) Proprietor: ABBOTT LABORATORIES, Abbott Park, Illinois 60064-3500 (US)
(72) Inventor: Devare, Sushil G., Northbrook, IL 60062 (US); Suresh, Desai M., Libertyville, IL 60048 (US); Casey, James M., Gurnee, IL 60031 (US)
(74) Representative: Modiano, Guido, Dr.-Ing.

(56) References cited:
- EP-A- 0 001 931
- EP-A- 0 187 041
- EP-A- 0 199 301
- EP-A- 0 331 961
- EP-A- 0 400 245
- WO-A-88/03562
- GENE, vol. 45, no. 3, 1986, pages 317-325, Elsevier Science B.V. (Biomedical Division), Amsterdam (NL); K.A. KELLEY et al., pp. 317-325
- THE EMBO JOURNAL, vol. 6, no. 3, March 1987, Eynsham, Oxford (GB); H. WEBER et al., pp. 591-598
- NATURE, vol. 326, 16 April 1987; M. GUYADER et al., pp. 662-669

## Description

The present invention relates to a recombinant HIV (Human Immunodeficiency Virus) antigen. A recombinant antigen derived from the molecular cloning and expression in a heterologous expression system of a synthetic DNA sequence of the various HIV antigens can be used as a reagent for the detection of antibodies and antigen in body fluids from individuals exposed to various HIV isolates.

The nucleotide sequence of the proviral genome has been determined for several HIV isolates, including HIV-1 strains HTLV-III (Ratner et al., *Nature* (1985) 313:277); ARV-2 (Sanchez-Pescador et al., *Science* (1985) 227:484); LAV (Wain-Hobson et al., *Cell* (1985) 40:9); and CDC-451 (Desai et al., *Proc. Natl*. *Acad. Sci*. *USA* (1986) 83:8380). The nucleotide sequence of the HIV-2 ROD isolate was reported by Guyader et al. *(Nature* (1987) 326:662).

HIV antigens have been obtained from the virus grown in tissue culture, or from a molecularly cloned genomic fragment expressed in heterologous hosts such as Escherichia coli. The tissue culture derived virus involves the cumbersome and often difficult process of growing virus infected cells in stringent sterile conditions. Further, the virus derived from tissue culture is infectious, and, therefore is hazardous to the health of individuals involved in propagation and purification. The expression of molecularly cloned HIV genomic fragments overcomes the biohazard problem. Generally, an HIV genomic fragment from a single HIV isolate with mammalian codons is expressed in a heterologous system, such as, bacteria or yeast, and is limited to the use of available restriction sites present in the viral genome for cloning and expression.

It has been difficult to obtain expression in heterologous systems of some of the HIV proteins, such as the HIV-1 envelope antigen gp41. Several researchers have tried deleting the hydrophobic regions of the HIV-1 gp41 to increase expression levels. UK Patent Application GB 2188639 discloses an HTLV-III gag/env gene protein wherein the env fragment of the DNA sequence deleted codons corresponding to the first hydrophobic region of the gp41 protein. U.S. Patent No. 4,753,873 discloses a peptide fragment that is encoded by a nucleotide sequence wherein the nucleotides coding for a first and second hydrophobic region of HTLV-III gp41 are deleted.

Poor expression can be the result of many factors, including the specific nucleic acid sequence of the gene to be expressed, the mammalian codons of the gene sequence to be expressed may not be efficiently transcribed and translated in a particular heterologous system, and the secondary structure of the transcribed messenger RNA. The use of synthetic DNA fragments can increase expression in heterologous systems.

### SUMMARY OF THE INVENTION

A recombinant antigen which is derived from the molecular cloning and expression of a synthetic DNA sequence in heterologous hosts is provided. A synthetic DNA sequence coding for the recombinant antigen of the invention is further provided. The synthetic DNA sequence selected for expression of various HIV antigens is based on the amino acid sequence of either a single isolate or several isolates, optimized for expression in Escherichia coli by specific codon selection. The synthetic DNA sequence gives higher expression of the particular antigen encoded. This antigen can be substituted for viral antigens derived from tissue culture for use as diagnostic and therapeutic reagents.

The present invention can be utilized to synthesize full length HIV transmembrane envelope gene using bacterial codons. Another aspect of the invention involves the linkage of sequences which are poorly expressed as individual proteins, to sequences which are expressed with high efficiency. The combination of the sequence of the entire coding region of a gene of one virus with coding sequences of another gene from a different virus to produce a fusion protein can be achieved. The fusion proteins thus expressed have a unique advantage of antigenic epitopes of two viral antigens.

The present invention includes a full length synthetic gene (FSG) for HIV-1 transmembrane glycoprotein (TMP).

### DESCRIPTION OF THE DRAWINGS

Fig. 1 illustrates the alignment of the TMP fragment encoding amino acid residue nos. 552-668 of HIV-1 with the sequences of the four different isolates used to derive the amino acid sequence of BS2-10.

Fig. 2 illustrates the assembly of 16 oligonucleotides to form the synthetic TMP fragment of Fig. 1, and its cloning into pUC18, designated BS2-10.

Fig. 3 illustrates the DNA and amino acid sequence of FSG, indicating the restriction sites and subfragments used for assembly.

Fig. 4 is a comparison of the amino acid sequence used to develop the synthetic HIV-1 envelope gene with known amino acid sequences of 13 independent isolates, indicating all linker-derived sequences (+) and amino acid substitutions (*).

Fig. 5 is a schematic diagram of the assembly and cloning of the major subfragments to form FSG in pUC18.

Fig. 6 is a schematic diagram of the cloning of FSG into lambda pL expression vectors to generate pSD301 and pSD302.

Figs. 7 illustrates the amino acid sequences of pSD301 and pSD302, indicating all linker-derived sequences (+) and amino acid substitutions (*).

Fig. 8 illustrates results of expression analysis of pSD301 and pSD302. A) Coomassie stained gel; B) Immunoblot using AIDS patients' sera.

### DETAILED DESCRIPTION OF THE INVENTION

Synthetic DNA fragments of the HIV genome can be synthesized based on their corresponding amino acid sequences. By comparing the particular region of interest between different isolates, a sequence can be selected which is different from any sequence that exists in nature, because the sequence is a compilation of the sequences from various isolates. For example, the synthetic HIV-1 envelope protein described in Example 1, is based on the amino acid sequence of four different HIV 1 isolates, namely, HTLV-IIIB, LAV-1, ARV-2 and CDC-451.

Other properties can be built into the sequence. For example, codons can be switched for optimal expression in bacteria or yeast, specific restriction sites can be introduced, and other restriction sites can be removed. In addition, the sequence should have specific restriction sites at both 5′ and 3′ ends of the fragment to facilitate cloning in a particular vector. Synthetic DNA fragments can be synthesized as follows: (1) select an unique protein sequence, (2) reverse translate to determine complementary DNA sequence, (3) optimize codons for bacterial or yeast expression, and (4) introduce and/or remove specific restriction sites.

Sixty-one distinct nucleotide codons code for 20 amino acids giving rise to the degeneracy in the genetic code. Researchers have reported the frequencies of codons used in nucleic acids for both eukaryotic and prokaryotic organisms. (Grantham et al., *Nucleic Acids Res.* [1980] 9:r43; Gouy et al., *Nucleic Acids Res.* [1982] 10:7055; Sharp et al., *Nucleic Acids Res.* [1986] 14:7737.) Sequences from the entire *E. coli* genome, with examples of sequences from the chromosome, transposons, and plasmids, have been analyzed. These sequences code for structural proteins, enzymes and regulatory proteins. Correlation has been shown between the degree of codon bias within a particular gene and the level of gene expression.

It is preferred that the same codon triplet for each particular amino acid of the synthetic DNA sequence be used. However, alternative codon(s) can be used to add or delete a particular restriction site. The sequence should include unique restriction sites which can be used to delete a specific fragment or sequence, or substitute a particular sequence in case of an error in the original synthesis.

Vector systems which can be used include plant, bacterial, yeast, insect, and mammalian expression systems. It is preferred that the codons are optimized for expression in the system used. The proteins and polypeptides provided by the invention, which are cloned and expressed in heterologous systems, as described above, can be used for diagnostic and therapeutic purposes.

A preferred expression system utilizes the lambda pL vector system. This expression system has the following features: (1) a strong lambda pL promoter, (2) a strong three-frame translation terminator rrnBt1, and (3) translation starts at an ATG codon, eight base pairs from the ribosome binding site located within an accessible Ncol restriction site.

Another advantage of the expression system of the present invention is that one can customize the synthetic DNA fragments so they contain specific DNA sequences which express proteins with desired amino acid sequences, and further allows one the capability of adding, at either the 5′ or 3′ end, other DNA sequences to facilitate the transfer of synthetic fragments into various vectors.

Additionally, the use of particular restriction sites at both ends of the fragment may also facilitate incorporation of the fragment into other sequences to generate fusion proteins, which can also be used as diagnostic and therapeutic reagents. For example, the HIV-1 gp41 sequence can be incorporated within or at the end of core/surface antigen of the hepatitis B viral sequence to generate a fusion protein which can be used in a single assay screening system for the detection of both AIDS and Hepatitis B in prospective blood donors. Alternatively, the assay can be used to track the course of a patient's infection.

Other proteins from any source, including bacterial, yeast, insect, plant or mammalian, can be used with the synthetic DNA fragments of the invention to produce fusion proteins. Those which are expressed efficiently in their respective expression systems are especially preferred because they can enhance the expression of the synthetic fragment of the fusion protein.

The synthetic DNA sequences of the present invention, derived from several HIV isolates and optimized for expression in *E. coli*, provides continuous availability and uniformity of HIV antigen preparations which will recognize test sera from individuals exposed to genetically distinguishable variant HIV isolates. The recombinant antigen expression is very stable since *E. coli* codons have been used for its synthesis. Moreover, the insertion of specific restriction sites allows addition, deletion, or substitution in important antigenic epitopes in the coding sequences, a property difficult to achieve when naturally occurring HIV sequences are utilized for expression. Construction of synthetic genes also allows the addition of protein sequences at either amino- or carboxyl- terminus of the gene thereby allowing the development of novel reagents. For example, a fusion gene can be produced comprising a fusion between HIV-1 core antigen and HIV-1 envelope synthetic gene. More specifically the envelope synthetic gene comprises the carboxyl-terminus HIV-1 gp120 sequence and the full length HIV-1 gp41. Similarly, the HIV-1 core antigen DNA sequence can be fused to the HIV-2 gp41 sequences, both of which can be expressed at high levels in heterologous host systems such as *E. coli.*

*E. coli* strains containing plasmids useful for constructs of the invention have been deposited at the American Type Culture Collection, Rockville, Maryland, on November 22, 1988, under the accession nos. ATCC 67855 (pSD301/RR1/pRK248.clts) and ATCC 67856 (pSD306/CAG456).

The following examples further describe the invention. The examples are not intended to limit the invention in any manner.

### Reagents and enzymes

Media such as Luria-Bertani (LB) and Superbroth II (Dri Form) were obtained from Gibco Laboratories Life Technologies, Inc., Madison, Wisconsin. Restriction enzymes, Klenow fragment of DNA polymerase I, T4 DNA ligase, T4 polynucleotide kinase, nucleic acid molecular weight standards- M13 sequencing system, X-gal (5-bromo-4-chloro-3-indonyl-β-D-galactoside), IPTG (isopropyl-β-D-thiogalactoside), glycerol, Dithiothreitol, 4-chloro-1-napthol were purchased from Boehringer Mannheim Biochemicals, Indianapolis, Indiana; or New England Biolabs, Inc., Beverly, Massachusetts; or Bethesda Research Laboratories Life Technologies, Inc., Gaithersburg, Maryland. Prestained protein molecular weight standards, acrylamide (crystallized, electrophoretic grade >99%); N-N′-Methylene-bis-acrylamide (BIS); N,N,N′,N′,-Tetramethylethylenediamine (TEMED) and sodium dodecylsulfate (SDS) were purchased from BioRad Laboratories, Richmond, California. Lysozyme and ampicillin were obtained from Sigma Chemical Co., St. Louis, Missouri. Horseradish peroxidase (HRPO) labeled secondary antibodies were obtained from Kirkegaard & Perry Laboratories, Inc., Gaithersburg, Maryland. Seaplaque agarose (low melting agarose) was purchased from FMC Bioproducts, Rockland, Maine.

T50E10 contained 50 mM Tris, pH 8:0, 10 mM EDTA; 1X TG contained 100 mM Tris, pH 7.5 and 10% glycerol; 2X SDS/PAGE loading buffer consisted of 15% glycerol, 5% SDS, 100 mM Tris base, 1M β-mercaptoethanol and 0.8% Bromophenol blue dye; TBS contained 50 mM Tris, pH 8.0, and 150 mM sodium chloride; Blocking solution consisted of 5% Carnation nonfat dry milk in TBS.

### Host cell cultures, DNA sources and vectors

*E. coli* JM103 cells, pUC8, pUC18, pUC19 and M13 cloning vectors were purchased from Pharmacia LKB Biotechnology, Inc., Piscataway, New Jersey; Competent Epicurean™ coli strains XL1-Blue and JM109 were purchased from Stratagene Cloning Systems, La Jolla, California. RR1 cells were obtained from Coli Genetic Stock Center, Yale University, New Haven, Connecticut; and *E. coli* CAG456 cells from Dr. Carol Gross, University of Wisconsin, Madison, Wisconsin. Vector pRK248.clts was obtained from Dr. Donald R. Helinski, University of California, San Diego, California.

### General methods

All restriction enzyme digestions were performed according to suppliers' instructions. At least 5 units of enzyme were used per microgram of DNA, and sufficient incubation was allowed to complete digestions of DNA. Standard procedures were used for mini cell lysate DNA preparation, phenol-chloroform extraction, ethanol precipitation of DNA, restriction analysis of DNA on agarose, and low melting agarose gel purification of DNA fragments (Maniatis et al., *Molecular Cloning. A Laboratory Manual* [New York: Cold Spring Harbor, 1982]). Plasmid isolations from *E. coli* strains used the alkali lysis procedure and cesium chloride-ethidium bromide density gradient method (Maniatis et al., supra). Standard buffers were used for T4 DNA ligase and T4 polynucleotide kinase (Maniatis et al., supra).

### EXAMPLES

### Example 1

### Cloning strategy of codon-optimized synthetic HIV-1 envelope protein

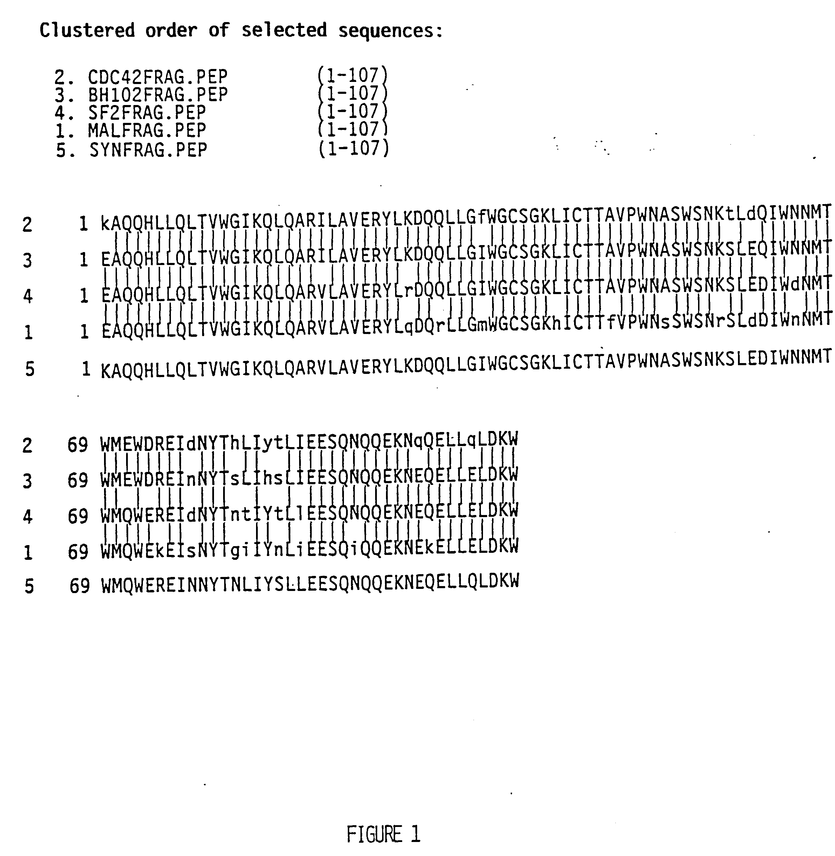

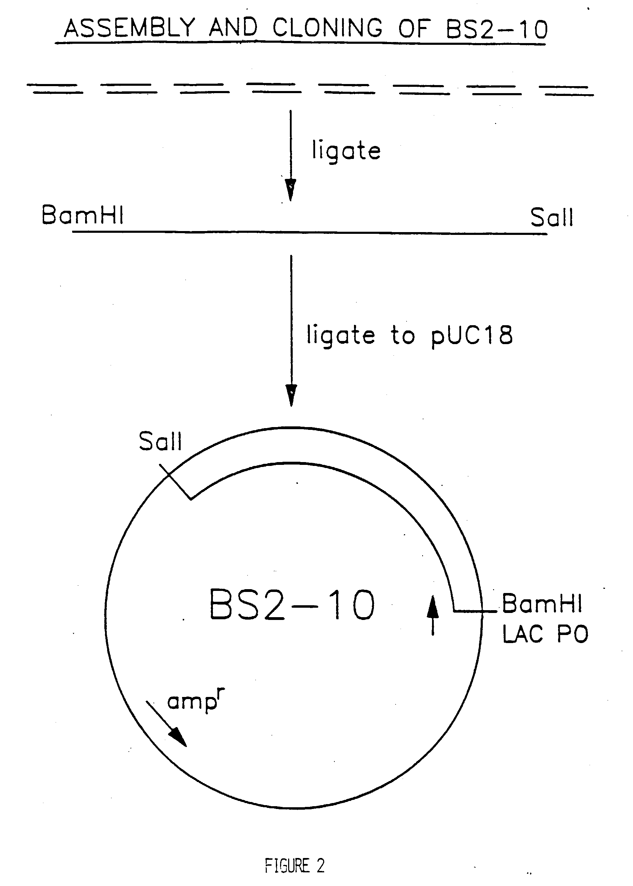

In order to develop a synthetic gene encoding the HIV-1 envelope glycoprotein and fragments thereof, the amino acid sequences of four independent HIV-1 viral isolates designated as HTLV-IIIB (BH102), LAV-1 (MAL), ARV-2 (SF), and CDC-451 (CDC42) were compared. A unique amino acid sequence from the four isolates (Fig. 1) was selected to derive a fragment with amino acid residues nos. 552-668 (numbering by Ratner et al., supra). This fragment contained nine amino acid substitutions (8%) as compared to the HTLV-IIIB (BH102) isolate. This amino acid sequence was reverse translated using codons optimized to facilitate high level expression in *E. coli.* The ambiguous nucleotides remaining in the second and/or third base of the codon were assigned to facilitate molecular cloning, and the addition, substitution, or deletion of sequences. The DNA sequence was then subdivided into eight double stranded fragments with unique 6 bp overhangs to direct specific annealing. The sixteen individual oligonucleotides were synthesized on Applied Biosystem 380A DNA synthesizer using methods and reagents recommended by the manufacturer. These purified oligonucleotides were annealed and ligated together to assemble the entire fragment which was digested with BamHI and SalI, ligated into pUC18 and transformed into *E. coli* JM103 cells. A clone designated BS2-10 (Fig. 2) was isolated, restriction mapped and its DNA sequence confirmed using the Sanger dideoxy chain termination method (Sanger et al., *J. Mol. Biol*. (1982) 162:729).

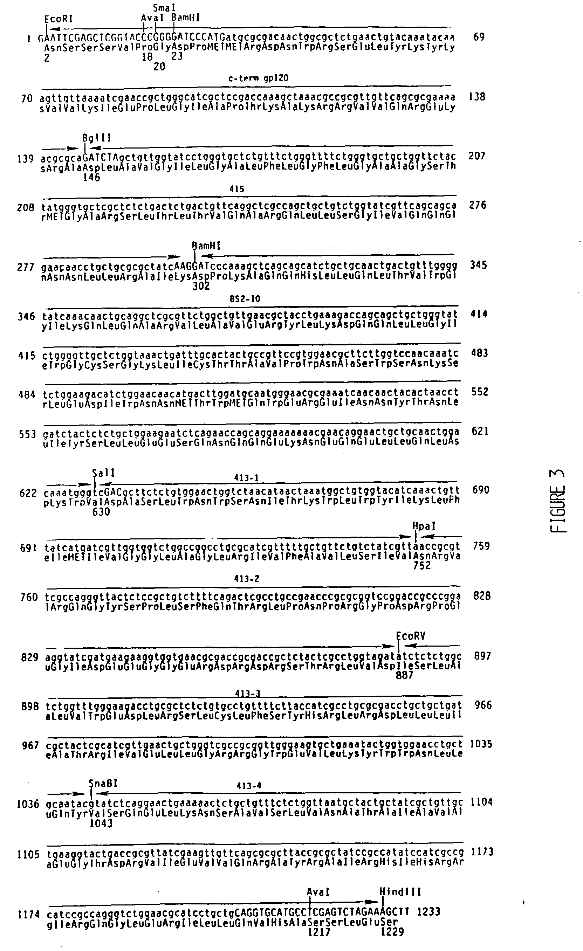

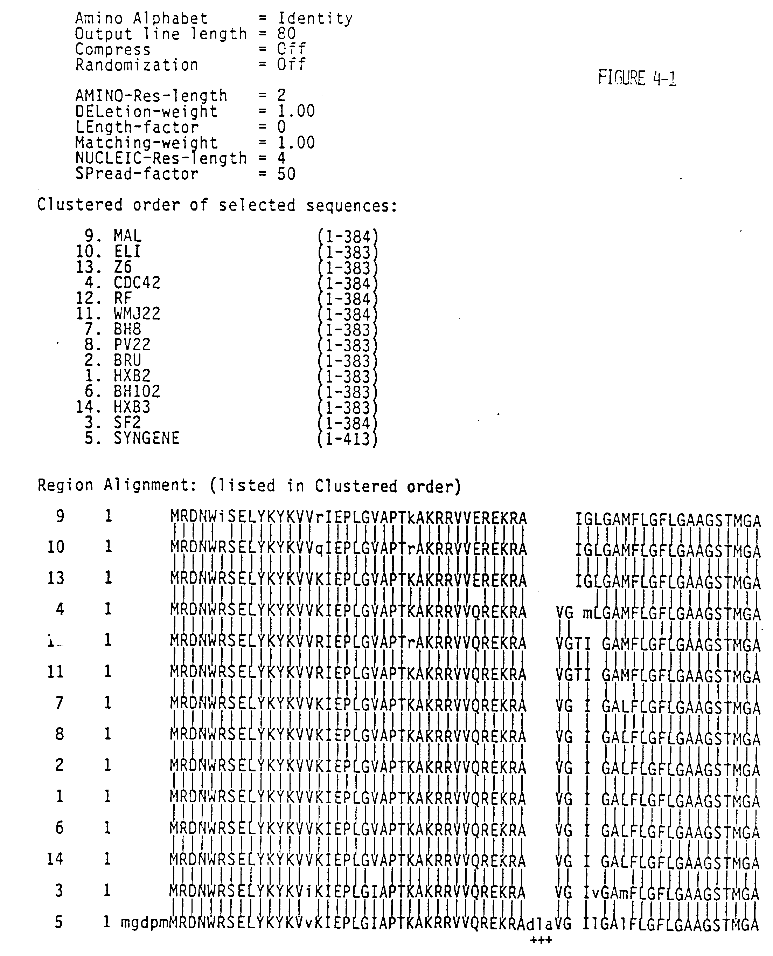

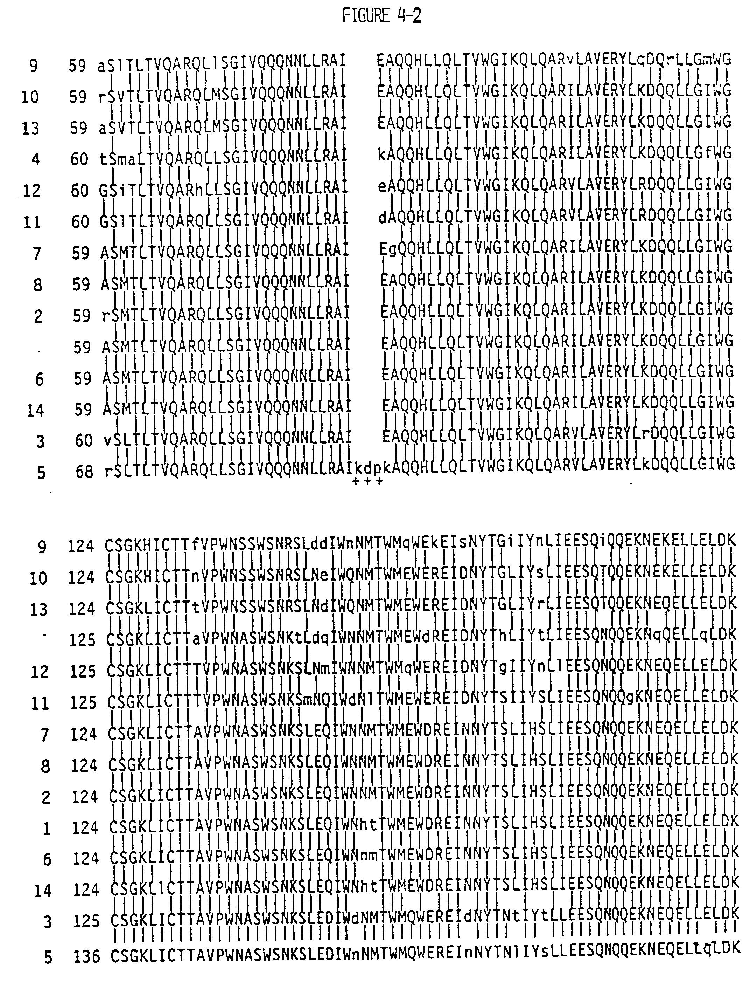

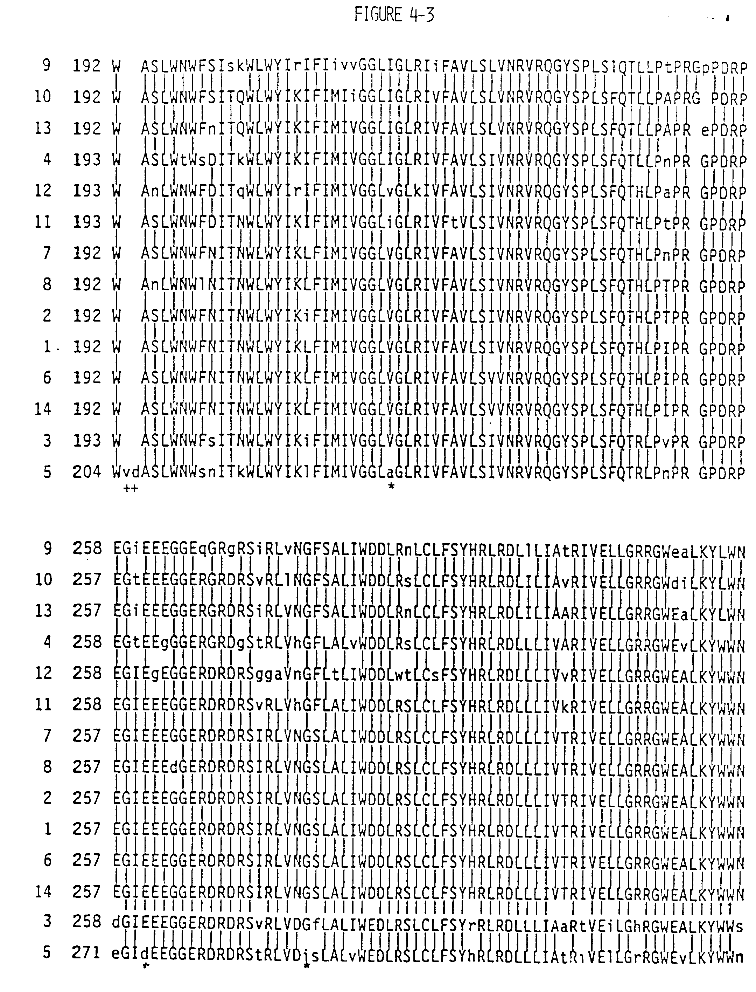

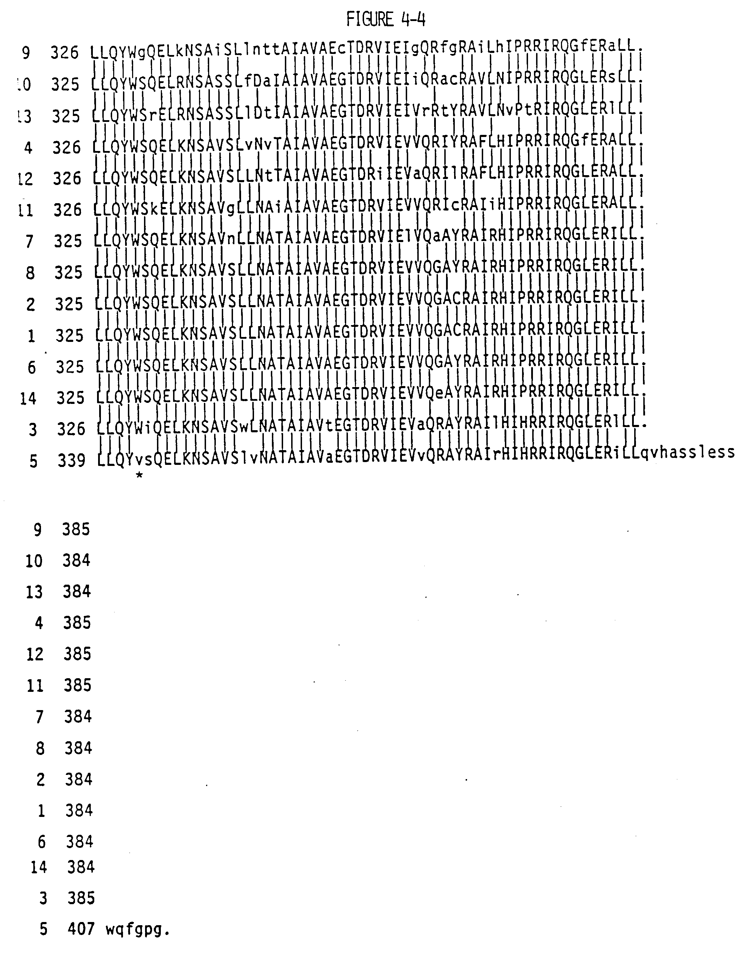

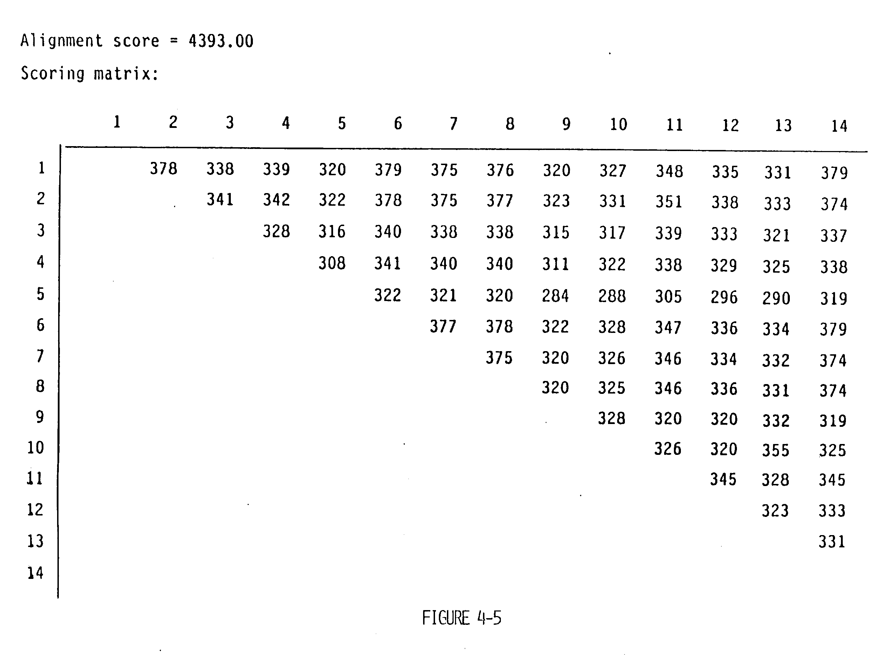

In order to establish that clone BS2-10 expressed this unique HIV-1 transmembrane protein (TMP) fragment, the BS2-10/JM103 culture was grown at 37°C in 50 ml Luria Broth, in a 250 ml Erlenmeyer flask. When the cultures reached an OD600 of 0.3-0.5, IPTG was added to a final concentration of 1 mM to induce expression. Samples (1.5 ml) were removed at 1 hr intervals, and the cells were pelleted and resuspended to an OD600 of 10.0 in 2X SDS/PAGE loading buffer. Aliquots (15 µl) of the prepared samples were loaded on a 15% SDS/PAGE gel, and the proteins were separated and then electrophoretically transferred to nitrocellulose for immunoblotting. The nitrocellulose sheet containing the transferred proteins was incubated with Blocking Solution for one hour and incubated overnight at 4° C with AIDS patients' sera diluted in TBS containing 5% *E. coli* JM103 lysate. The nitrocellulose sheet was washed three times in TBS, then incubated with HRPO-labeled goat anti-human IgG, diluted in TBS containing 10% fetal calf sera. The nitrocellulose was washed three times with TBS and the color was developed in TBS containing 2 mg/ml 4-chloro-1-napthol, 0.02% hydrogen peroxide and 17% methanol. Clone BS2-10 demonstrated a strongly immunoreactive band with AIDS patients' sera indicating that the synthetic HIV-1 TMP fragment was expressed in *E. coli.* In order to assemble the full length HIV-1 transmembrane protein, as well as the extreme carboxyl-terminal 37 amino acids of gp120, the amino acid sequences of the four isolates described previously were compared to each other to derive a unique amino acid sequence for this gene. After this unique amino acid sequence was reverse translated using codons optimized for *E. coli* expression, the ambiguous nucleotides were assigned as previously described. The full length synthetic HIV-1 envelope gene (FSG) was divided into six additional subfragments. The complete DNA and amino acid sequence of FSG is shown in Fig. 3, indicating the restriction sites and subfragments used for assembly. Fig. 4 is a comparison of the amino acid sequence used to develop the synthetic HIV-1 envelope gene with known amino acid sequences of 13 independent isolates reported in the Los Alamos HIV Data Bank (Meyers et al., *Human Retroviruses and AIDS* (1987), Los Alamos National Laboratory). The Genalign program of Intelligenetics was used to align these sequences, and the alignment demonstrates that FSG (designated SYNGENE in Fig. 4) retains substantial overall sequence homology compared to other known isolates. Alignment parameters and alignment scores of the individual sequences are also shown.

### Synthesis and cloning of subfragments

The subfragments located downstream from BS2-10, designated 413-1 through 413-4, were synthesized along with additional sequences containing a BamHI restriction site at the 5′ end and a HindIII restriction site at the 3′ end to facilitate molecular cloning and DNA sequence analysis of the individual subfragments. The subfragments located upstream of BS2-10 were also synthesized with additional sequences containing restriction sites useful for cloning and DNA sequence analysis. The subfragment encoding the carboxyl-terminal gp120 amino acid sequence, designated c-term gp120, contained EcoRI and BamHI restriction sites on the 5′ end and BgIII and SmaI restriction sites on the 3′ end. Similarly, subfragment 415 contained a BgIII site on the 5′ end and BgIII and BamHI restriction sites on the 3′ end. With the exception of the c-term gp120 subfragment, in which both strands were synthesized as described for BS2-10, the remaining subfragments of FSG were synthesized by a method utilizing the Klenow fragment of DNA polymerase I. In this method, oligonucleotides comprising opposite strands of a particular subfragment, which contained ten complementary bases, were synthesized and annealed. The second complementary strand was then filled in by the Klenow fragment of DNA polymerase I in the presence of the four deoxynucleotides in a manner similar to that described by Sanger et al., supra, for DNA sequencing. The resulting double-stranded subfragment was then digested with the appropriate restriction enzymes and cloned into pUC vectors to confirm the DNA sequence, as previously described. Subfragments 413-1 through 413-4 were cloned into pUC18 using the BamHI and HindIII restriction sites common to all. Subfragment c-term gp120 was cloned into pUC8 using the EcoRI and SmaI restriction sites. Subfragment 415 was cloned into the plasmid containing c-term gp120 at the BgIII restriction site and screened for proper orientation by restriction mapping. The plasmid DNAs for all subfragments were prepared by the cesium chloride buoyant density gradient method and the individual DNA sequences were confirmed directly from the double-stranded template (Hattori et al., *Nucl. Acid Res.* (1985) 13:7813).

### Assembly and cloning of FSG

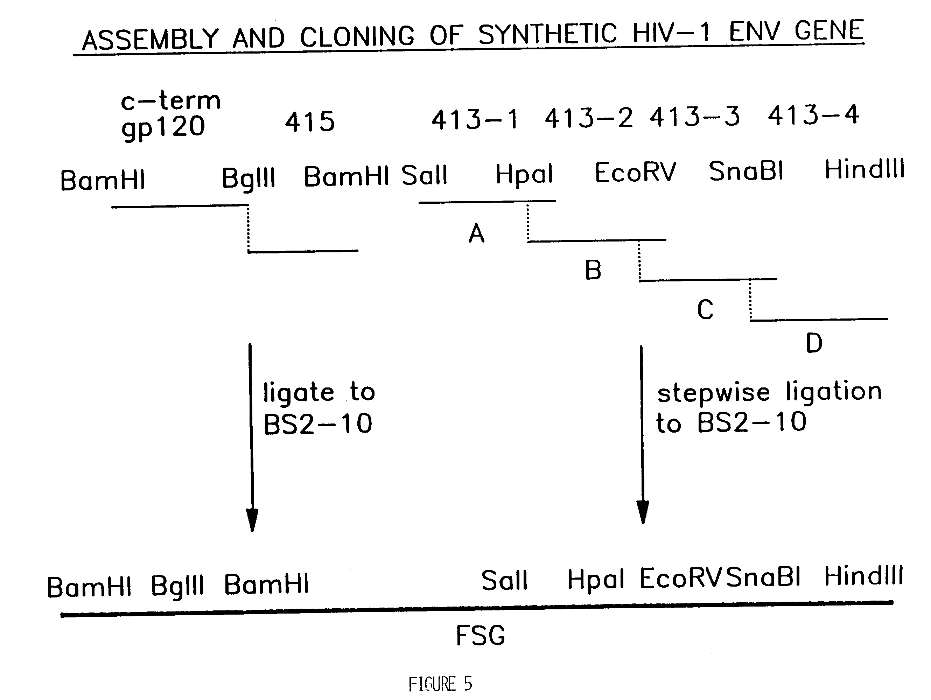

Subfragments located downstream from BS2-10 were cloned in a stepwise fashion utilizing unique internal restriction sites at the 5′ end and a common HindIII site at the 3′ end. For example, subfragment 413-1 was cloned into BS2-10 at the SalI and HindIII restriction sites to generate clone BS2-10A, into which 413-2 was inserted at the HpaI and HindIII restriction sites to generate clone BS2-108. Similarly, subfragments 413-3 and 413-4 were added using unique EcoRV and SnaBI restriction sites, respectively. The two subfragments located upstream of clone BS2-10, having been cloned together in pUC8, were ligated to BS2-10 as a BamHI fragment. Fig. 5 shows the cloning method used to assemble the synthetic HIV-1 envelope gene in pUC18. The final clone, designated FSG, was restriction mapped to confirm the proper orientation of the BamHI-BamHI fragment.

### Example 2

### Cloning and expression of FSG in lambda pL Vector Systems

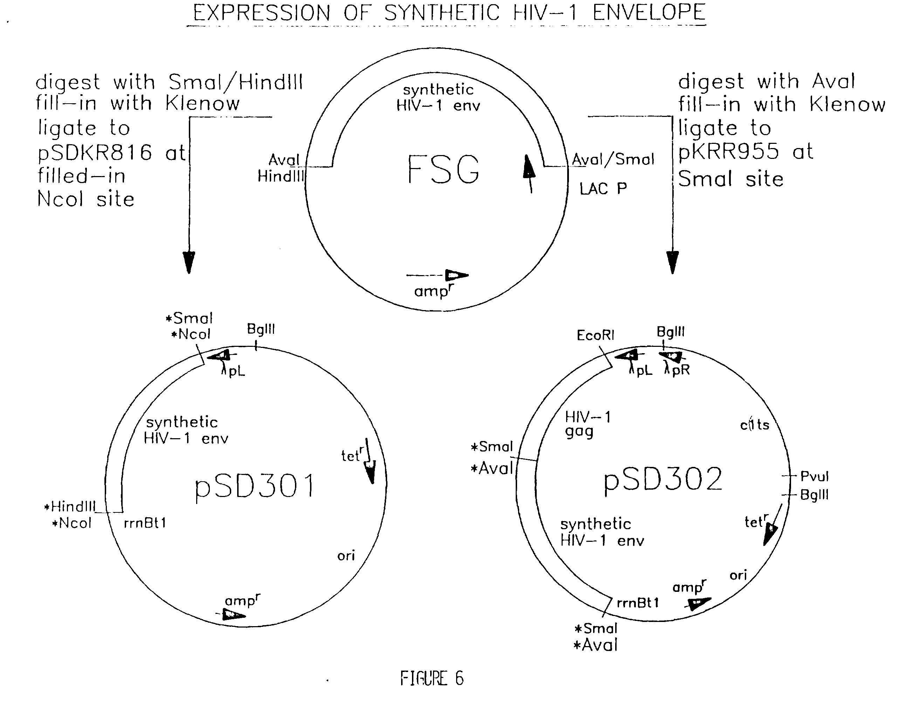

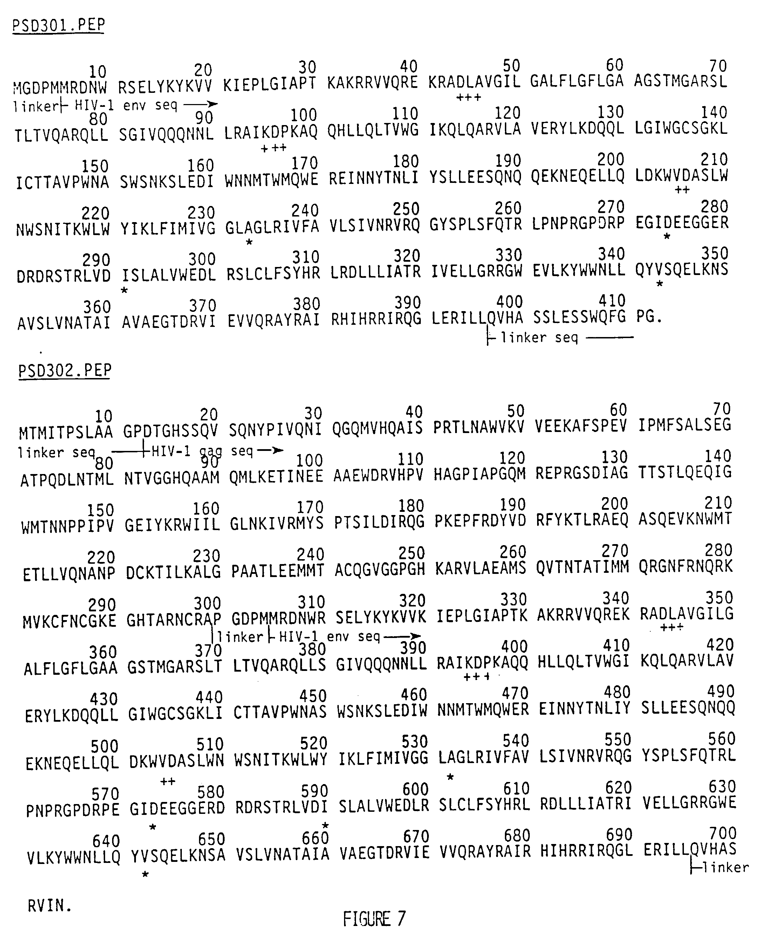

Expression analysis of FSG was carried out in vector systems utilizing the strong lambda pL promoter and the temperature sensitive cl repressor gene (Benard et al., *Gene* (1979) 5:59). The specific vectors used in these analyses are derivatives of pBR322, containing a lambda pL promoter and a synthetic Shine-Dalgamo sequence, followed by restriction sites used for cloning various genes of interest. In addition, these vectors contain the strong three-frame translation terminator rrnBt1. Vector pSDKR816 contains a Ncol restriction site which provides an ATG start codon optimally spaced from the start of transcription. Fig. 6 schematically presents the cloning of FSG into pSDKR816 to generate clone pSD301. Briefly, FSG was digested with Hindlll and Smal, the ends were made blunt by filling in with the Klenow fragment of DNA polymerase I, and the 1209 bp fragment was purified and ligated into pSDKR816 at the Ncol site filled in with the Klenow fragment of DNA polymerase I. After transformation into *E. coli* RR1 cells containing the clts gene on the compatible vector pRK248, a clone with FSG in the proper orientation was isolated by restriction mapping and designated pSD301. The specific amino acid sequence encoded by pSD301 is presented in Fig. 7 indicating all linker derived sequences (+) and all amino acid substitutions within the HIV-1 envelope sequences not yet identified in any published sequence (*).

Additionally, FSG was cloned as a fusion to the HIV-1 gag protein (amino acid residue nos. 121-407, numbering by Ratner et al., supra) which is highly expressed under control of the lambda pL promoter in vector pKRR955. FSG was digested with Aval, the ends were made blunt by filling in with the Klenow fragment of DNA polymerase I, and the 1199 bp fragment was purified and ligated into pKRR955 at the Smal restriction site to form an HIV-1 gag/synthetic env fusion protein (Fig. 6). After transformation into *E. coli* pRK248.clts/RR1 cells, a clone containing FSG in the proper orientation was identified by restriction mapping and designated pSD302. The specific amino acid sequence of this fusion protein is presented in Fig. 7 indicating all linker derived sequences, HIV-1 gag sequences, and HIV-1 envelope sequences as previously described.

Fifty ml cultures of pSD301 and pSD302 in *E. coli* pRK248.clts/RR1 cells were grown in Superbroth II media at 30°C to an OD600 of 0.5, at which time the cultures were shifted to 42°C to inactivate the temperature sensitive cl repressor and thereby induce expression off the lambda pL promoter. Two samples (2.0 ml each) were removed at 1 hr intervals. Sample preparation was as follows.

The cells were pelleted, then resuspended in either 1X TG buffer or T50E10 buffer. An equal volume of 2X SDS/PAGE loading buffer was added to the 1X TG suspended cells to produce the whole lysate. The sample resuspended in T50E10 was sonicated eight times for 30 seconds each, at a power setting of 10 watts, using the microtip provided with the Vibra Cell Sonicator (Sonics and Materials, Inc., Danbury, CT). The sonicated sample was then centrifuged to remove the insoluble fraction which was resuspended in the original starting volume of T50E10. An equal volume of 2X SDS/PAGE loading buffer was added to both the sonicated soluble and insoluble fractions, which together with the whole cell lysate, were boiled for 5 min, centrifuged to remove any remaining insoluble material, and aliquots (15µl) were separated on duplicate 12.5% SDS/PAGE gels. Proteins from one such gel were electrophoretically transferred to nitrocellulose for immunoblotting with AIDS patients' sera, as previously described. The second gel was fixed in a solution of 50% methanol, 10% acetic acid for twenty minutes at room temperature, and then stained with 0.25% Coomassie blue dye in a solution of 50% methanol, 10% acetic acid for 30 minutes. Destaining was carried out using a solution of 10% methanol, 7% acetic acid for 3-4 hr, or until a clear background was obtained.

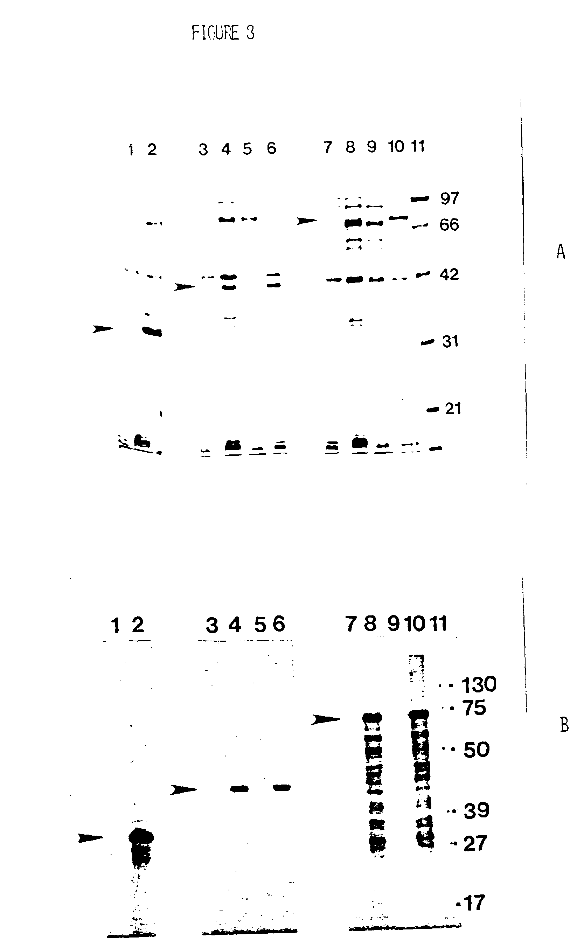

Fig. 8 presents the expression of pSD301 and pSD302 prior to (T0) and four hours post (T4) induction, analyzed by Coomassie blue staining (Fig. 8A) and immunoblotting (Fig. 8B). Samples were pKRR955 (T0 whole cell lysate [lane 1], T4 whole cell lysate [lane 2]); pSD301 (T0 whole cell lysate [lane 3], T4 whole cell lysate [lane 4], T4 sonicated soluble fraction [lane 5], and T4 sonicated insoluble fraction [lane 6]); and pSD302 (T0 whole cell lysate [lane 7], T4 whole cell lysate [lane 8], T4 sonicated soluble fraction [lane 9], and T4 sonicated insoluble fraction [lane 10]). Molecular weight standards were run in lane 11. Arrows indicate the position of the induced proteins which are clearly visualized in both the whole cell lysate and the sonicated insoluble cell fraction by Coomassie blue staining (Fig. 8A). Lane 2 indicates that pKRR955 expressed the HIV-1 gag protein at a level greater than 25% of total cellular protein, lane 4 indicates that pSD301 expressed the synthetic HIV-1 envelope protein at a level of approximately 12% of total cellular protein, and lane 8 indicates that pSD302 expressed the HIV-1 gag/synthetic env fusion protein at a level of approximately 5% of total cellular protein. The expression levels obtained using FSG were significantly higher than those obtained using the corresponding native viral DNA sequences in similar pL vector systems. All three recombinant proteins were highly reactive with AIDS patients' sera (Fig. 8B). This data demonstrates that the synthetic HIV-1 envelope gene, including the hydrophobic region of the transmembrane protein, can be efficiently expressed in *E. coli,* and the expressed proteins are highly immunoreactive.

### Diagnostic utility of synthetic DNA derived HIV proteins

The HIV specific proteins overexpressed in E. *coli* were purified using procedures known in the art. The proteins expressed at high levels were immunogenic and were recognized by antibodies produced in HIV-infected individuals (see fig. 8). The HIV specific proteins derived from *E. coli* can be utilized in several immunoassay configurations, as described in CIP application U.S. Serial No. 020,282, filed February 27, 1987 by Dawson et al., which is hereby incorporated by reference. The parent application is EPO 86116854.0 (December 4, 1986). In a preferred configuration, HIV specific proteins were coated on solid support and incubated with test samples. The virus specific antibodies present in the test sample recognized and were bound to the HIV proteins on the solid support. The HIV specific antibodies were quanfitated by the use of goat anti-human immunoglobulin conjugated to HRPO.

Biological samples which are easily tested by the methods of the present invention include human and animal body fluids such as whole blood, serum, plasma, urine, saliva, stools, lymphocyte or cell culture preparations and purified and partially purified immunoglobulins. The polypeptide described herein can be used to determine the presence or absence of antibodies to HIV-1 antigen by assay methods known to those skilled in the art.

One such assay involves:
a) coating a solid support with the polypeptide disclosed herein;
b) contacting the coated solid support with the biological sample to form an antibody polypeptide complex;
c) removing unbound biological sample from the solid support;
d) contacting the complex on the solid support with a labeled immunoglobulin specific for the antibody; and
e) detecting the label to determine the presence or absence of HIV-1 antibodies in the biological sample.

A second assay method involves:
a) coating a solid support with the polypeptide disclosed herein;
b) contacting the coated solid support with the biological sample and the homologous polypeptides conjugated to a label;
c) removing unbound biological sample and unbound labeled polypeptide; and
d) detecting the label to determine the presence or absence of HIV-1 antibodies in the biological sample.

Solid supports which can be used in such immunoassays include wells of reaction trays, test tubes, beads, strips, membranes, filters, microparticles or other solid supports which are well known to those skilled in the art. Enzymatic, radioisotopic, fluorescent, chemiluminescent and colloidal particle labels can be used in the aforementioned assays. Furthermore, hapten/labeled anti-hapten systems such as a biotin/labeled anti-biotin system can be utilized in the inventive assays. Both polyclonal and monoclonal antibodies are useful as reagents, and IgG as well as IgM class HIV antibodies may be used as solid support or labeled reagents.

It is evident from the foregoing examples that one skilled in the art could clone together specific subfragments of the synthetic genes constructed to generate new synthetic genes that would have the same characteristics as those illustrated herein. For example, the c-term gp120 subfragment, BS2-10 and subfragment 413-1 can be cloned together to produce synthetic gene products useful as diagnostic and therapeutic reagents for AIDS.

## Claims (Claims for the following Contracting State(s): AT, BE, CH, DE, FR, GB, GR, IT, LI, NL, SE)

1. A polypeptide comprising an amino acid sequence represented by the following:

MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.

2. The polypeptide of Claim 1 produced by E. coli.

3. A fusion polypeptide comprising a polypeptide as in Claim 1, in which the polypeptide is fused to a prokaryotic or eukaryotic protein.

4. The fusion polypeptide of Claim 3 wherein said prokaryotic or eukaryotic protein is the E. coli enzyme CKS.

5. A synthetic gene comprising a DNA sequence represented by the following:

ATGGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
CCGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA

6. The synthetic gene of Claim 5 coding for the polypeptide of Claim 1.

7. A fusion polypeptide comprising a polypeptide as in Claim 1, in which the polypeptide is fused to a HIV gag protein.

8. The fusion polypeptide of Claim 7 wherein said HIV gag protein is an HIV-1 gag protein comprising an amino acid sequence represented by the following:

DTGHSSQVSQNYPIVQNIQGQMVHQAISPRTLNAWVKVVEEKAFSPEVIPMFSALSEGATPQDLNTMLNT
VGGHQAAMQMLKETINEEAAEWDRVHPVHAGPIAPGQMREPRGSDIAGTTSTLQEQIGWMTNNPPIPVGE
IYKRWIILGLNKIVRMYSPTSILDIRQGPKEPFRDYVDRFYKTLRAEQASQEVKNWMTETLLVQNANPDC
KTILKALGPAATLEEMMTACQGVGGPGHKARVLAEAMSQVTNTATIMMQRGNFRNQRKMVKCFNCGKEGH
TARNCRA

9. A method for detecting antibodies to HIV antigens in an individual which comprises the steps of:
a) obtaining a sample of a body fluid from the individual:
b) incubating said body fluid with said polypeptide of Claim 1;
c) incubating said body fluid with a labeled antibody to immunoglobulin; and
d) detecting said label and determining therefrom the presence or absence of antibodies to HIV antigens.

10. A method for detecting antibodies to HIV antigens in an individual which comprises the steps of:
a) obtaining a sample of a body fluid from the individual:
b) incubating said body fluid with said polypeptide of Claim 1;
c) incubating said body fluid with a labeled antigen; and
d) detecting said label determining therefrom the presence or absence of antibodies to HIV antigens.

## Claims (Claims for the following Contracting State(s): ES)

1. A method for detecting antibodies to HIV antigens in an individual which comprises the steps of:
a) obtaining a sample of a body fluid from the individual:
b) incubating said body fluid with a polypeptide comprising an amino acid sequence represented by the following: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
c) incubating said body fluid with a labeled antibody to immunoglobulin; and
d) detecting said label and determining therefrom the presence or absence of antibodies to HIV antigens.

2. A method for detecting antibodies to HIV antigens in an individual which comprises the steps of:
a) obtaining a sample of a body fluid from the individual:
b) incubating said body fluid with a polypeptide comprising an amino acid sequence represented by the following: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
c) incubating said body fluid with a labeled antigen; and
d) detecting said label and determining therefrom the presence or absence of antibodies to HIV antigens.

3. A method for producing a polypeptide comprising an amino acid sequence represented by the following: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
 said method comprising the molecular cloning and expression in a heterologous host of a synthetic gene comprising a DNA sequence represented by the following:

ATGGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
CCGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA

4. The method of Claim 3 wherein the heterologous host is E. coli.

5. The method of Claim 3 wherein said DNA sequence is fused to a second DNA sequence coding for a prokaryotic or eukaryotic protein whereby a fusion protein is expressed comprising said amino acid sequence fused to a prokaryotic or eukaryotic protein.

6. The method of Claim 5 wherein said prokaryotic or eukaryotic protein is the E. coli enzyme CKS.

7. The method of Claim 3 wherein said DNA sequence is fused to a second DNA sequence coding for a HIV gag protein whereby a fusion protein is expressed comprising said amino acid sequence fused to a HIV gag protein.

8. The method of Claim 7 wherein said second DNA sequence codes for a HIV-1 gag protein comprising an amino acid sequence represented by the following:

DTGHSSQVSQNYPIVQNIQGQMVHQAISPRTLNAWVKVVEEKAFSPEVIPMFSALSEGATPQDLNTMLNT
VGGHQAAMQMLKETINEEAAEWDRVHPVHAGPIAPGQMREPRGSDIAGTTSTLQEQIGWMTNNPPIPVGE
IYKRWIILGLNKIVRMYSPTSILDIRQGPKEPFRDYVDRFYKTLRAEQASQEVKNWMTETLLVQNANPDC
KTILKALGPAATLEEMMTACQGVGGPGHKARVLAEAMSQVTNTATIMMQRGNFRNQRKMVKCFNCGKEGH
TARNCRA

## Patentansprüche (Patentansprüche für folgende(n) Vertragsstaat(en): AT, BE, CH, LI, DE, FR, GB, GR, IT, NL, SE)

1. Polypeptid, das eine Aminosäuresequenz umfaßt, die wie folgt dargestellt werden kann:

MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.

2. Polypeptid nach Anspruch 1, das von E. coli hergestellt wird.

3. Fusionspolypeptid, das ein Polypeptid nach Anspruch 1 umfaßt, bei dem das Polypeptid an ein prokaryontisches oder eukaryontischen Protein fusioniert ist.

4. Fusionspolypeptid nach Anspruch 3, wobei das prokaryontische oder eukaryontische Protein das E. coli-Enzym CKS ist.

5. Synthetisches Gen, das eine DNA-Sequenz umfaßt, die wie folgt dargestellt werden kann:

ATGGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
[?]CGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA.

6. Synthetisches Gen nach Anspruch 5, das für ein Polypeptid nach Anspruch 1 codiert.

7. Fusionspolypeptid, das ein Polypeptid nach Anspruch 1 umfaßt, bei dem das Polypeptid an ein HIV-gag-Protein fusioniert ist.

8. Fusionspolypeptid nach Anspruch 7, worin das HIV-gag-Protein ein HIV-1-gag-Protein ist, das eine Aminosäuresequenz umfaßt, die durch folgendes dargestellt werden kann:

DTGHSSQVSQNYPIVQNIQGQMVHQAISPRTLNAWVKVVEEKAFSPEVIPMFSALSEGATPQDLNTMLNT
VGGHQAAMQMLKETINEEAAEWDRVHPVHAGPIAPGQMREPRGSDIAGTTSTLQEQIGWMTNNPPIPVGE
IYKRWIILGLNKIVRMYSPTSILDIRQGPKEPFRDYVDRFYKTLRAEQASQEVKNWMTETLLVQNANPDC
KTILKALGPAATLEEMMTACQGVGGPGHKARVLAEAMSQVTNTATIMMQRGNFRNQRKMVKCFNCGKEGH
TARNCRA

9. Verfahren zum Nachweis von Antikörpern gegen HIV-Antigene bei einem Individuum, das folgende Schritte umfaßt:
a) Erhalt einer Probe einer Körperflüssigkeit des Individuums;
b) Inkubation der Körperflüssigkeit mit dem Polypeptid nach Anspruch 1;
c) Inkubation der Körperflüssigkeit mit einem markierten Antikörper gegen Immunglobulin; und
d) Nachweis der Markierung und daraus Bestimmung der Anwesenheit oder Abwesenheit von Antikörpern gegen HIV-Antigene.

10. Verfahren zum Nachweis von Antikörpern gegen HIV-Antigene bei einem Individuum, das folgende Schritte umfaßt:
a) Erhalt einer Probe von Körperflüssigkeit des Individuums;
b) Inkubation der Körperflüssigkeit mit dem Polypeptid nach Anspruch 1;
c) Inkubation der Körperflüssigkeit mit einem markierten Antigen; und
d) Nachweis der Markierung, wobei dadurch die Anwesenheit oder Abwesenheit von Antikörpern gegen HIV-Antigene bestimmt wird.

## Patentansprüche (Patentansprüche für folgende(n) Vertragsstaat(en): ES)

1. Verfahren zum Nachweis von Antikörpern gegen HIV-Antigene bei einem Individuum, das folgende Schritte umfaßt:
a) Erhalt eine Probe einer Körperflüssigkeit des Individuums,
b) Inkubation der Körperflüssigkeit mit einem Polypeptid, das eine Aminosäuresequenz umfaßt, die durch folgendes dargestellt werden kann: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG
c) Inkubation der Körperfüssigkeit mit einem markierten Antikörper gegen Immunglobulin; und
d) Nachweis der Markierung und daraus Bestimmung der Anwesenheit oder Abwesenheit von Antikörpern gegen HIV-Antigene.

2. Verfahren zum Nachweis von Antikörpern gegen HIV-Antigene bei einem Individuum, das folgende Schritte umfaßt:
a) Erhalt einer Probe von Körperflüssigkeit des Individuums,
b) Inkubation der Körperflüssigkeit mit einem Polypeptid, das eine Aminosäuresequenz umfaßt, die wie folgt dargestellt werden kann: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG
c) Inkubation der Körperflüssigkeit mit einem markierten Antigen, und
d) Nachweis der Markierung und daraus Bestimmung der Anwesenheit oder-Abwesenheit von Antikörpern gegen HIV-Antigene.

3. Verfahren zur Herstellung eines Polypeptids, das eine Aminosäuresequenz umfaßt, die durch folgendes dargestellt werden kann: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG
 wobei das Verfahren das molekulare Klonen und die Expression eines synthetischen Gens in einem heterologen Wirt umfaßt, wobei das Gen eine DNA-Sequenz umfaßt, die wie folgt dargestellt werden kann:

ATGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
CCGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA,

4. Verfahren nach Anspruch 3, wobei der heterologe Wirt E. Coli. ist.

5. Verfahren nach Anspruch 3, wobei die DNA-Sequenz an eine zweite DNA-Sequenz fusioniert ist, die für ein prokaryontisches oder eukaryontisches Protein codiert, wobei ein Fusionsprotein exprimiert wird, das die Aminosäuresequenz umfaßt, die an ein prokaryontisches oder eukaryontisches Protein fusioniert ist.

6. Verfahren nach Anspruch 5, wobei das prokaryontische oder eukaryontische Protein das E. Coli-Enzym CKS ist.

7. Verfahren nach Anspruch 3, wobei die DNA-Sequenz an eine zweite DNA-Sequenz fusioniert ist, die für ein HIV-gag-Protein codiert, wodurch ein Fusionsprotein exprimiert wird, das die Aminosäuresequenz umfaßt, die an ein HIV-gag-Protein fusioniert ist.

8. Verfahren nach Anspruch 7, wobei die zweite DNA-Sequenz für ein HIV-1-gag-Protein codiert, das eine Aminosäuresequenz umfaßt, die wie folgt dargestellt werden kann:

DTGHSSQVSQNYPIVQNIQGQMVHQAISPRTLNAWVKVVEEKAFSPEVIPMFSALSEGATPQDLNTMLNT
VGGHQAAMQMLKETINEEAAEWDRVHPVHAGPIAPGQMREPRGSDIAGTTSTLQEQIGWMTNNPPIPVGE
IYKRWIILGLNKIVRMYSPTSILDIRQGPKEPFRDYVDRFYKTLRAEQASQEVKNWMTETLLVQNANPDC
KTILKALGPAATLEEMMTACQGVGGPGHKARVLAEAMSQVTNTATIMMQRGNFRNQRKMVKCFNCGKEGH
TARNCRA.

## Revendications (Revendications pour l'(les) Etat(s) contractant(s) suivant(s): AT, BE, CH, LI, DE, FR, GB, GR, IT, NL, SE)

1. Polypeptide comprenant une séquence d'aminoacides représentée par la séquence suivante:

MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.

2. Polypeptide selon la revendication 1, produit par *E. coli.*

3. Polypeptide de fusion comprenant un polypeptide selon la revendication 1, dans lequel le polypeptide est fusionné avec une protéine eucaryote ou procaryote.

4. Polypeptide de fusion selon la revendication 3, dans lequel ladite protéine eucaryote ou procaryote est l'enzyme CKS d'*E. coli.*

5. Gène synthétique comprenant une séquence d'ADN représentée par la séquence suivante:

ATGGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
CCGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA

6. Gène synthétique selon la revendication 5, codant pour le polypeptide de la revendication 1.

7. Polypeptide de fusion comprenant un polypeptide selon la revendication 1, dans lequel le polypeptide est fusionné avec une protéine gag de HIV.

8. Polypeptide de fusion selon la revendication 7, dans lequel ladite protéine gag de HIV est une protéine gag de HIV-1 comprenant une séquence d'aminoacides représentée par la séquence suivante:

DTGHSSQVSQNYPIVQNIQGQMVHQAISPRTLNAWVKVVEEKAFSPEVIPMFSALSEGATPQDLNTMLNT
VGGHQAAMQMLKETINEEAAEWDRVHPVHAGPIAPGQMREPRGSDIAGTTSTLQEQIGWMTNNPPIPVGE
IYKRWIILGLNKIVRMYSPTSILDIRQGPKEPFRDYVDRFYKTLRAEQASQEVKNWMTETLLVQNANPDC
KTILKALGPAATLEEMMTACQGVGGPGHKARVLAEAMSQVTNTATIMMQRGNFRNQRKMVKCFNCGKEGH
TARNCRA

9. Procédé de détection d'anticorps dirigés contre des antigènes de HIV chez un individu, qui comprend les étapes selon lesquelles:
a) on prélève un échantillon d'un liquide corporel de l'individu;
b) on met ledit liquide corporel à incuber avec ledit polypeptide de la revendication 1;
c) on met ledit liquide corporel à incuber avec un anticorps anti-immunoglobuline marqué; et
d) on détecte ledit marqueur et on en déduit la présence ou l'absence d'anticorps dirigés contre les antigènes de HIV.

10. Procédé de détection d'anticorps dirigés contre des antigènes de HIV chez un individu, qui comprend les étapes selon lesquelles:
a) on prélève un échantillon d'un liquide corporel de l'individu;
b) on met ledit liquide corporel à incuber avec ledit polypeptide de la revendication 1;
c) on met ledit liquide corporel à incuber avec un antigène marqué; et
d) on détecte ledit marqueur et on en déduit la présence ou l'absence d'anticorps dirigés contre les antigènes de HIV.

## Revendications (Revendications pour l'(les) Etat(s) contractant(s) suivant(s): ES)

1. Procédé de détection d'anticorps dirigés contre des antigènes de HIV chez un individu, qui comprend les étapes selon lesquelles:
a) on prélève un échantillon d'un liquide corporel de l'individu;
b) on met ledit liquide corporel à incuber avec un polypeptide comprenant une séquence d'aminoacides représentée par la séquence suivante: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
c) on met ledit liquide corporel à incuber avec un anticorps anti-immunoglobuline marqué; et
d) on détecte ledit marqueur et on en déduit la présence ou l'absence d'anticorps dirigés contre les antigènes de HIV.

2. Procédé de détection d'anticorps dirigés contre des antigènes de HIV chez un individu, qui comprend les étapes selon lesquelles:
a) on prélève un échantillon d'un liquide corporel de l'individu;
b) on met ledit liquide corporel à incuber avec un polypeptide comprenant une séquence d'aminoacides représentée par la séquence suivante: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEQELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
c) on met ledit liquide corporel à incuber avec un antigène marqué; et
d) on détecte ledit marqueur et on en déduit la présence ou l'absence d'anticorps dirigés contre les antigènes de HIV.

3. Procédé de production d'un polypeptide comprenant une séquence d'aminoacides représentée par la séquence suivante: 
MGDPMMRDNWRSELYKYKVVKIEPLGIAPTKAKRRVVQREKRADLAVGILGALFLGFLGAAGSTMGARSL
TLTVQARQLLSGIVQQQNNLLRAIKDPKAQQHLLQLTVWGIKQLQARVLAVERYLKDQQLLGIWGCSGKL
ICTTAVPWNASWSNKSLEDIWNNMTWMQWEREINNYTNLIYSLLEESQNQQEKNEOELLQLDKWVDASLW
NWSNITKWLWYIKLFIMIVGGLAGLRIVFAVLSIVNRVRQGYSPLSFQTRLPNPRGPDRPEGIDEEGGER
DRDRSTRLVDISLALVWEDLRSLCLFSYHRLRDLLLIATRIVELLGRRGWEVLKYWWNLLQYVSQELKNS
AVSLVNATAIAVAEGTDRVIEVVQRAYRAIRHIHRRIRQGLERILLQVHASSLESSWQFGPG.
 ledit procédé comprenant le clonage moléculaire et l'expression dans un hôte hétérologue d'un gène synthétique comprenant une séquence d'ADN représentée par la séquence suivante:

ATGGGGGATCCCATGATGCGCGACAACTGGCGCTCTGAACTGTACAAATACAAAGTTGTTAAAATCGAAC
CGCTGGGCATCGCTCCGACCAAAGCTAAACGCCGCGTTGTTCAGCGCGAAAAACGCGCAGATCTAGCTGT
TGGTATCCTGGGTGCTCTGTTTCTGGGTTTTCTGGGTGCTGCTGGTTCTACTATGGGTGCTCGCTCTCTG
ACTCTGACTGTTCAGGCTCGCCAGCTGCTGTCTGGTATCGTTCAGCAGCAGAACAACCTGCTGCGCGCTA
TCAAGGATCCCAAAGCTCAGCAGCATCTGCTGCAACTGACTGTTTGGGGTATCAAACAACTGCAGGCTCG
CGTTCTGGCTGTTGAACGCTACCTGAAAGACCAGCAGCTGCTGGGTATCTGGGGTTGCTCTGGTAAACTG
ATTTGCACTACTGCCGTTCCGTGGAACGCTTCTTGGTCCAACAAATCTCTGGAAGACATCTGGAACAACA
TGACTTGGATGCAATGGGAACGCGAAATCAACAACTACACTAACCTGATCTACTCTCTGCTGGAAGAATC
TCAGAACCAGCAGGAAAAAAACGAACAGGAACTGCTGCAACTGGACAAATGGGTCGACGCTTCTCTGTGG
AACTGGTCTAACATAACTAAATGGCTGTGGTACATCAAACTGTTTATCATGATCGTTGGTGGTCTGGCCG
GCCTGCGCATCGTTTTTGCTGTTCTGTCTATCGTTAACCGCGTTCGCCAGGGTTACTCTCCGCTGTCTTT
TCAGACTCGCCTGCCGAACCCGCGCGGTCCGGACCGCCCGGAAGGTATCGATGAAGAAGGTGGTGAACGC
GACCGCGACCGCTCTACTCGCCTGGTAGATATCTCTCTGGCTCTGGTTTGGGAAGACCTGCGCTCTCTGT
GCCTGTTTTCTTACCATCGCCTGCGCGACCTGCTGCTGATCGCTACTCGCATCGTTGAACTGCTGGGTCG
CCGCGGTTGGGAAGTGCTGAAATACTGGTGGAACCTGCTGCAATACGTATCTCAGGAACTGAAAAACTCT
GCTGTTTCTCTGGTTAATGCTACTGCTATCGCTGTTGCTGAAGGTACTGACCGCGTTATCGAAGTTGTTC
AGCGCGCTTACCGCGCTATCCGCCATATCCATCGCCGCATCCGCCAGGGTCTGGAACGCATCCTGCTGCA
GGTGCATGCCTCGAGTCTAGAAAGCTCATGGCAATTCGGGCCCGGGTAA

4. Procédé selon la revendication 3, dans lequel l'hôte hétérologue est *E*. *coli.*

5. Procédé selon la revendication 3, dans lequel ladite séquence d'ADN est fusionnée avec une seconde séquence d'ADN codant pour une protéine procaryote ou eucaryote, grâce à quoi est exprimée une protéine de fusion comprenant ladite séquence d'aminoacides fusionnée avec une protéine procaryote ou eucaryote.

6. Procédé selon la revendication 5, dans lequel ladite protéine procaryote ou eucaryote est l'enzyme CKS d'*E*. *coli.*

7. Procédé selon la revendication 3, dans lequel ladite séquence d'ADN est fusionnée avec une seconde séquence d'ADN codant pour une protéine gag de HIV, grâce à quoi est exprimée une protéine de fusion comprenant ladite séquence d'aminoacides fusionnée avec une protéine gag de HIV.

8. Procédé selon la revendication 7, dans lequel ladite seconde séquence d'ADN code pour une protéine gag de HIV-1 comprenant une séquence d'aminoacides représentée par la séquence suivante:
